# DEMANDE DE BREVET EUROPEEN

(11) **EP 1 230 898 A1**
(43) Date de publication de la demande: **14.08.2002**
(21) Numéro de dépôt: 02290225.8
(22) Date de dépôt: 31.01.2002
(51) Int. Cl.: A61B 17/04, A61F 2/08

(54) **Ensemble d'ancrage de suture**

(30) Priorité: 07.02.2001 FR 0101647
(71) Demandeur: PHUSIS, 38330 Saint Ismier (FR)
(72) Inventeur: Huet-Olivier, Jacqueline, 38330 Saint-Ismier (FR); Boileau, Pascal, 06200 Nice (FR)
(74) Mandataire: Geismar, Thierry

(57) **Abrégé**

L'invention a pour objet un système implantable (16) pour l'ancrage de brins de suture (2) à l'intérieur d'un tunnel osseux (3), ledit système comprenant un dispositif implantable (1) et une pièce complémentaire (17), ledit dispositif étant formé d'une seule pièce réalisée en matériau biorésorbable et comprend :
- une gaine (4) agencée pour recevoir au moins un brin de suture (2) ; et
- une tête (5) disposée à une extrémité proximale (6) de ladite gaine (4) et s'étendant radialement depuis la paroi extérieure (7) de celle-ci ;
ladite pièce étant destinée à être introduite dans la gaine (4), de sorte à, dans une première position, laisser libre le déplacement des brins de suture (2) à l'intérieur de la gaine (4) et, dans une deuxième position, être apte à bloquer ce déplacement.

L'invention concerne également un kit d'ancrage comprenant un tel système.

## Description

L'invention concerne un système implantable pour l'ancrage de brins de suture à l'intérieur d'un tunnel osseux et un kit d'ancrage comprenant un tel système.

Elle s'applique typiquement à la fixation temporaire d'un tissu mou, par exemple une structure tendineuse ou musculaire, sur un os à l'aide de brins de suture.

Dans un exemple particulier, l'invention s'applique à la réparation et à la fixation de la coiffe des rotateurs, c'est à dire de la structure tendineuse située au niveau de l'épaule, sur l'humérus d'un patient humain souffrant d'une pathologie de ladite coiffe.

On connaît déjà des méthodes dans lesquelles les brins de suture sont passés dans la coiffe avant d'être amarrés soit directement en réalisant des points trans-osseux soit indirectement en passant la suture dans l'oeilleton d'une ancre préalablement fixée à l'os.

La fixation ainsi réalisée présente un certain nombre d'inconvénients notamment en ce qui concerne sa qualité.

En particulier, le contact direct qui existe entre les brins de suture et l'os conduit à un effet « fil à couper le beurre », c'est à dire à une pénétration des brins dans le pont osseux cortical par cisaillement, qui provoque un relâchement de l'effort de fixation.

Cet effet est d'autant plus important que la qualité osseuse est faible, et donc notamment chez les patients âgés qui présentent une tête humérale ostéoporotique.

La fixation indirecte par ancre peut également se révéler défaillante en raison de la faible densité de l'os spongieux dans laquelle elle est placée.

En outre, la fixation sous arthroscopie est souvent compliquée en raison des difficultés de réalisation et de serrage des noeuds entre brins de suture.

L'invention vise notamment à remédier à ces inconvénients en prévoyant un système implantable pour l'ancrage de brins de suture à l'intérieur d'un tunnel osseux qui évite le contact direct entre les brins de suture et l'os tout en étant facilement implantable et adaptable aux différentes géométries du tunnel osseux.

En outre, le système de l'invention ne nécessite pas la réalisation de noeuds entre brins de suture, ce qui est bien adaptée pour son implantation sous arthroscopie, notamment à l'aide du kit proposé.

A cet effet, et selon un premier aspect, l'invention propose un système implantable pour l'ancrage de brins de suture à l'intérieur d'un tunnel osseux, ledit système comprenant un dispositif implantable et une pièce complémentaire, ledit dispositif étant formé d'une seule pièce réalisée en matériau biorésorbable et comprend :
- une gaine agencée pour recevoir au moins un brin de suture ; et
- une tête disposée à une extrémité proximale de ladite gaine et s'étendant radialement depuis la paroi extérieure de celle-ci ;
ladite pièce étant destinée à être introduite dans la gaine, de sorte à, dans une première position, laisser libre le déplacement des brins de suture à l'intérieur de la gaine et, dans une deuxième position, être apte à bloquer ce déplacement.

Selon un deuxième aspect, l'invention propose un kit d'ancrage de brins de suture à l'intérieur d'un tunnel osseux, comprenant un tel système implantable et un dispositif d'implantation dudit système.

D'autres objets et avantages de l'invention apparaîtront au cours de la description qui suit en référence aux dessins annexés.

La figure 1 représente, en perspective, un mode de réalisation d'un dispositif implantable.

La figure 2 représente, en perspective, un mode de réalisation d'une pièce complémentaire formant avec le dispositif de la figure 1 un système implantable.

La figure 3 représente, en coupe et de façon schématique, le dispositif de la figure 1 implanté dans un tunnel osseux.

La figure 4 représente, en perspective et de façon schématique, la fixation d'une coiffe des rotateurs sur l'humérus à l'aide de trois dispositifs de la figure 1, la fixation étant réalisée par des points de suture trans-dispositif.

La figure 5 représente, en perspective, un système implantable comprenant le dispositif de la figure 1 et la pièce complémentaire de la figure 2, avant introduction de ladite pièce dans ledit dispositif.

La figure 6 représente, en perspective, le système implantable de la figure 6 dans lequel la pièce complémentaire est dans sa deuxième position.

La figure 7 représente, en perspective, un kit d'ancrage de brins de suture comprenant le système des figures 5 et 6 et un dispositif d'implantation dudit système, ledit système étant monté sur ledit dispositif pour permettre son implantation.

La figure 8 est une coupe longitudinale du kit représenté sur la figure 7.

La figure 9 représente, en perspective, une pièce d'actionnement de la pièce complémentaire.

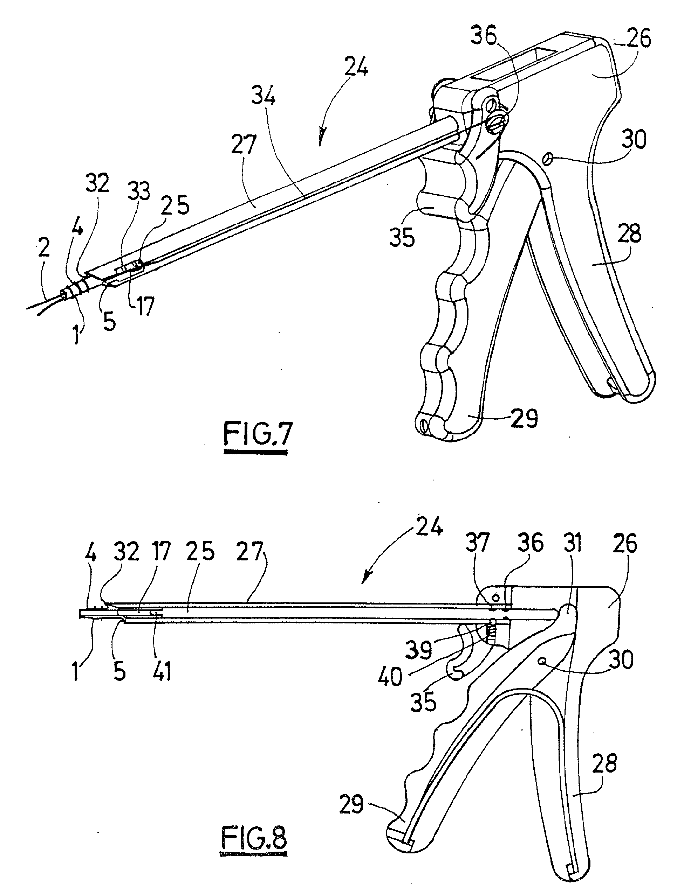

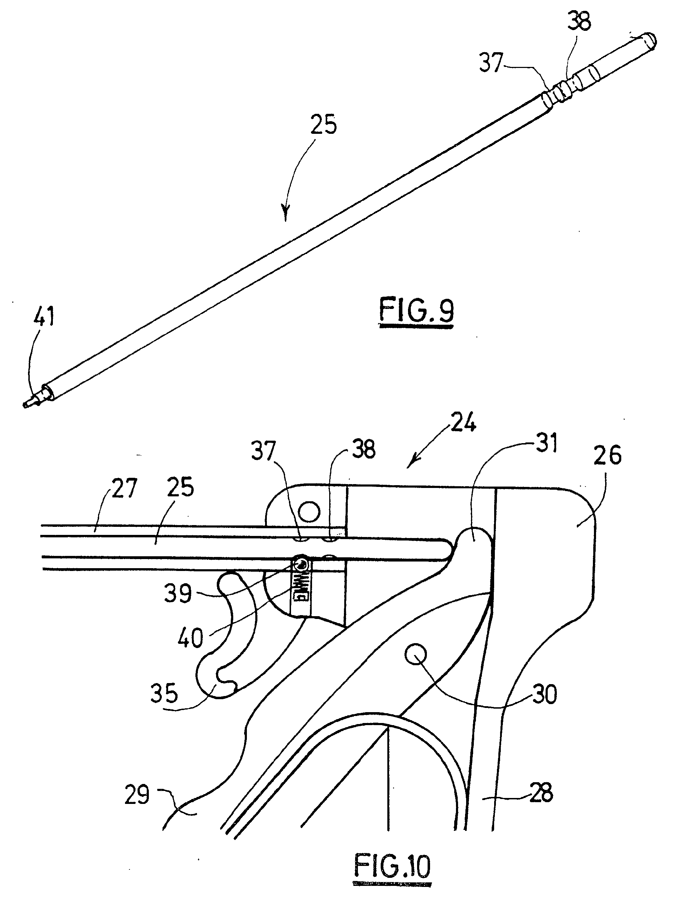

Le figure 10 représente, en coupe et de façon partielle, la disposition de la pièce de la figure 9 dans le dispositif d'implantation des figures 7 et 8.

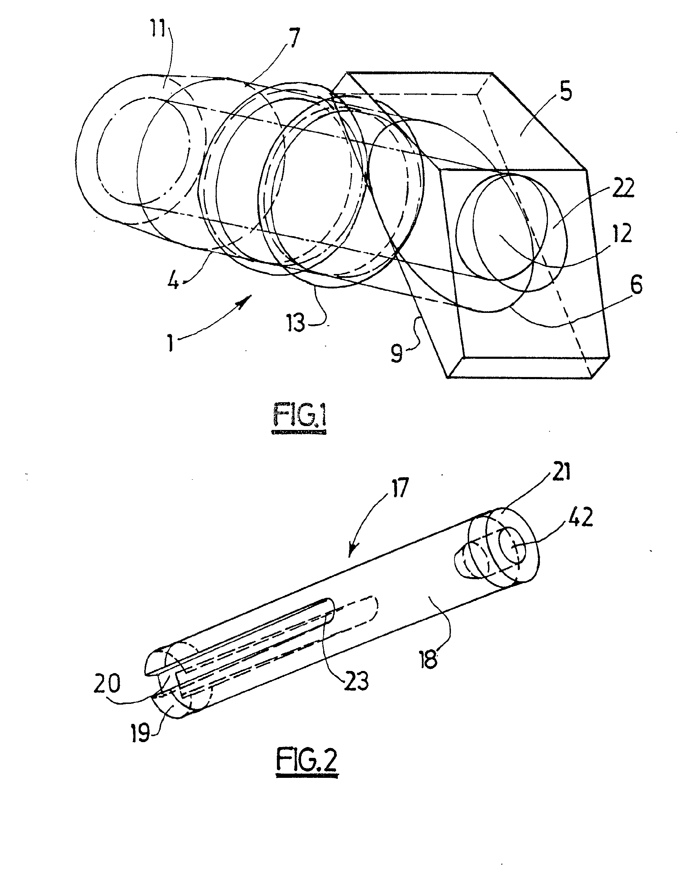

En relation avec la figure 1, on décrit un dispositif implantable 1 pour l'ancrage de brins de suture 2 à l'intérieur d'un tunnel osseux 3 qui comprend :
- une gaine 4 agencée pour recevoir au moins un brin de suture 2 ; et
- une tête 5 disposée à une extrémité proximale 6 de ladite gaine 4 et s'étendant radialement depuis la paroi extérieure 7 de celle-ci.

Dans la description, les termes « proximal » et « distal » sont définis par rapport au sens d'implantation du dispositif 1 dans le tunnel osseux 3.

Le dispositif 1 est formé en matériau biorésorbable et est réalisé en une seule pièce notamment par moulage par injection.

Suivant une réalisation, et pour qu'il présente une bonne aptitude au moulage, le matériau biorésorbable comprend les polymères de grande pureté chimique, de masse moléculaire supérieure à environ 250 000 et de faible polydispersité, par exemple inférieur à 2.

Le matériau biorésorbable peut comprendre les stéréocopolymères des acides L- et D-Lactique, les homopolymères de l'acide L-Lactique, les copolymères de l'acide lactique et d'un comonomère compatible tel que les dérivés d'alpha-hydroxy-acides, ainsi que les dérivés et/ou mélanges de ces produits, par exemple synthétisé suivant le procédé décrit dans la demande de brevet FR-2 745 005 issue de la demanderesse.

Dans un exemple particulier, le matériau biorésorbable comprend 98% d'acide L-lactique et 2% de D-lactique, sa masse moléculaire moyenne est comprise entre 300 000 et 400 000 et sont indice de polydispersité est égal à 1,8.

Du fait de la disposition des brins de suture 2 dans la gaine 4 et de la présence de la tête 5 s'étendant radialement au niveau de la zone où l'effort de serrage est le plus important, le dispositif 1 permet d'éviter les contacts destructifs entre les brins 2 et l'os 8.

Par ailleurs, la tête 5, de par les différents angles que sa surface inférieure 9 (c'est à dire la face destinée à venir en contact avec l'os 8) peut former avec la gaine 4, permet d'adapter le dispositif 1 aux différentes géométries du tunnel osseux 3.

En effet, l'angle formé entre le tunnel osseux 3 préalablement percé et la surface de la corticale osseuse 10 peut être variable en fonction des contraintes géométriques du lieu d'implantation et, pour une bonne stabilité du dispositif 1, la surface inférieure 9 de la tête 5 doit venir en contact quasi plan avec la surface osseuse 10.

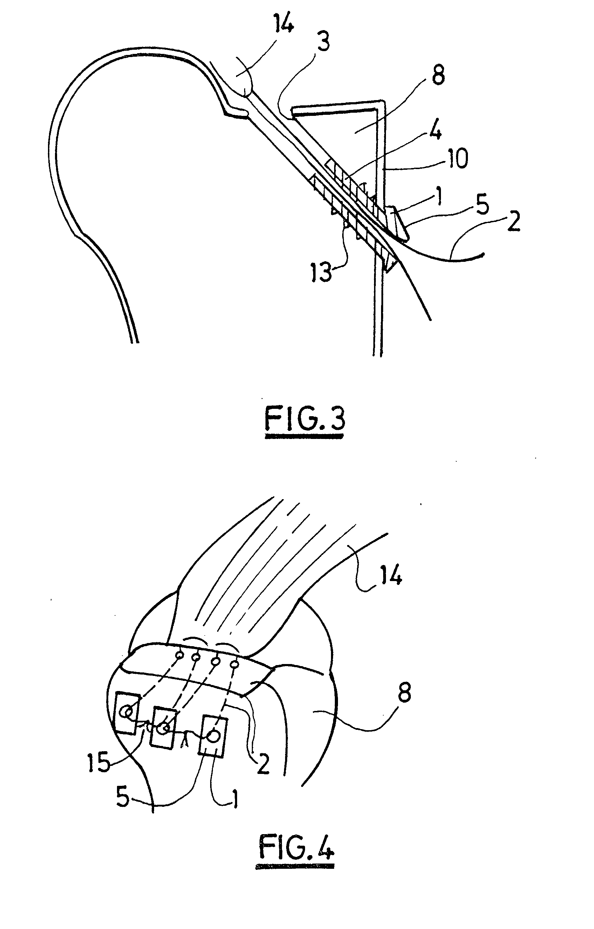

Dans un premier exemple (voir figure 3) et lorsque cet angle est sensiblement égale à 45°, le dispositif 1 peut être réalisé de sorte que la face inférieure 9 de la tête 5 forme un angle sensiblement égal à 45° avec l'axe longitudinal de la gaine 4.

Dans un deuxième exemple (non représenté) et lorsque cet angle est sensiblement égale à 90°, le dispositif 1 peut être réalisé de sorte que la face inférieure 9 de la tête 5 forme un angle sensiblement égal à 90° avec l'axe longitudinal de la gaine 4.

En outre, la tête 5 peut être de forme rectangulaire (voir figures) afin de limiter l'encombrement du dispositif 1 sur l'os 8 tout en assurant une bonne stabilité de l'implant.

En variante et pour permettre une meilleure solidarisation entre le dispositif 1 et l'os 8 dans lequel il est implanté, la paroi extérieure 7 de la gaine 4 comprend des moyens d'ancrage du dispositif 1 dans le tunnel osseux 3.

Suivant une réalisation (voir figure 1), la gaine 4 est formée d'une pièce annulaire de diamètre extérieur sensiblement égal ou légèrement inférieur à celui du tunnel osseux 3 et dont l'extrémité distale 11 est de forme tronconique de sorte à faciliter son introduction dans le tunnel osseux 3.

En outre, la pièce 4 possède une ouverture 12 de section sensiblement circulaire qui la traverse de part en part selon son axe longitudinal de sorte à être traversée par les brins de suture 2.

Les moyens d'ancrage peuvent alors être formés d'au moins une sur-épaisseur 13 en forme d'anneau qui s'étend radialement sur la surface externe 7 de la pièce annulaire et dont le diamètre extérieur est sensiblement égal ou légèrement supérieur à celui du tunnel osseux 3.

Lors de l'implantation (voir figure 3), le dispositif 1 est introduit en force dans le tunnel osseux 1 jusqu'à ce que la surface inférieure 9 de la tête 5 vienne en contact quasi-planaire avec la surface 10 de l'os cortical. Les brins de sutures 2 sont alors reçus dans la gaine 4 du dispositif 1 pour pouvoir coulisser librement.

La figure 4 représente la fixation d'une coiffe 14 des rotateurs sur l'humérus 8 à l'aide de trois dispositifs 1 implantés dans trois tunnels osseux 3 différents. Dans cet exemple, deux brins de suture 2 sont préalablement associés à la coiffe 14 avec quatre extrémités libres. Les deux extrémités extérieures sont respectivement disposées dans les deux dispositifs 1 extérieurs et les deux autres extrémités sont disposées dans le dispositif 1 central, la fixation est alors réalisée par des points de suture 15 trans-dispositif entre extrémités de brins de suture 2 adjacentes.

On décrit ci-dessous un mode de réalisation d'un système implantable 16 comprenant un dispositif 1 tel que décrit ci-dessus et une pièce complémentaire 17 qui permet de bloquer le déplacement des brins de suture 2 à l'intérieur de la gaine 4 de sorte à s'affranchir de la réalisation des points de suture 15.

La pièce complémentaire 17 est destinée à être introduite dans la gaine 4 de sorte que, dans une première position, elle laisse libre le déplacement des brins de suture 2 à l'intérieur de la gaine 4 et, dans une deuxième position, elle soit apte à bloquer ce déplacement.

Ainsi, dans la première position, les brins de suture 2 peuvent être tendus afin d'assurer un bon contact entre la coiffe 14 et l'os 8 puis, dans la deuxième position, les brins de suture 2 sont bloquer en position sans avoir recourt à des points 15.

Selon un mode de réalisation (voir figure 2) la pièce complémentaire 17 est formée d'une tige cylindrique 18 dont la section est légèrement inférieure au diamètre intérieur de la gaine 4 et dont la partie distale 19 est munie de rainures 20 destinées à recevoir chacune un brin de suture 2, lesdites rainures 20 étant agencées pour d'une part séparer lesdits brins 2 et d'autre part guider leur coulissement à l'intérieur de la gaine 4.

A cet effet, la profondeur des rainures 20 est prévue pour être supérieure au diamètre des brins de suture 2.

Dans un exemple particulier, la partie distale 19 comprend autant de rainure 20 que de brins 2 destinés à être reçus dans la gaine 4, à savoir deux rainures 20 dans le mode de réalisation représenté sur la figure 2, lesdites rainures 20 s'étendant suivant la direction longitudinale et sur moins de la moitié de la longueur totale de la tige 18 de sorte à obtenir un bon compromis entre le coulissement et le blocage des brins 2.

En outre, une tête 21 peut être prévue sur l'extrémité proximale de la tige 18, ladite tête 21 étant destinée d'une part à participer au coincement des brins 2 lorsque la tige 18 est dans sa deuxième position et d'autre part à servir de butée lors du positionnement de la tige 18 dans ladite deuxième position.

Pour permettre une bonne complémentarité entre le dispositif 1 et la pièce complémentaire 17, l'ouverture proximale 22 de la gaine 4 et la tête 21 de la tige 18 sont de forme conique, le diamètre de la tête 21 étant supérieur à celui de l'ouverture proximale 22, l'angulation de la tête 21 étant sensiblement égale ou supérieure à celle de l'ouverture proximale 22.

En outre, la hauteur de la tête 21 peut être prévue pour être sensiblement égale ou inférieure à la profondeur du cône de l'ouverture proximale 22, la tête 21, lorsque la pièce 17 est dans sa deuxième position, ne dépassant ainsi pas du dispositif 1 afin de ne pas risquer de « blesser » le milieu environnant.

Dans un exemple particulier, les angulations de la tête 21 et de l'ouverture proximale 22 sont de l'ordre de 10°.

La pièce complémentaire 17 est également en matériau biorésorbable, identique ou non à celui formant le dispositif 1.

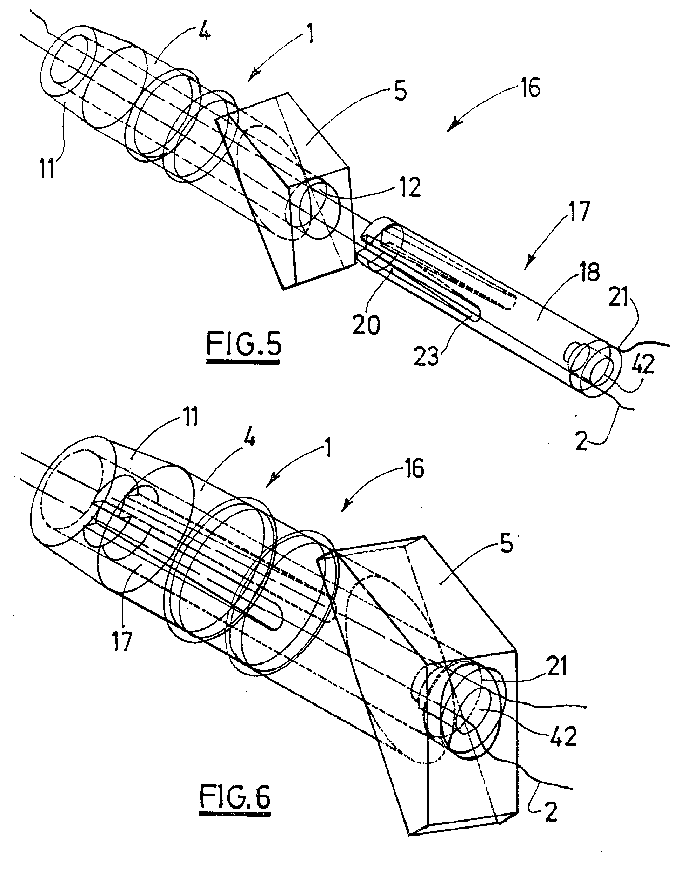

On décrit ci-dessous, en relation avec les figures 5 et 6, l'association de la pièce complémentaire 17 représentée sur la figure 2 dans le dispositif 1 représenté sur la figure 1.

La pièce 17 est introduite dans la gaine 4 avec les deux brins de suture 2 disposés respectivement dans une rainure 20 de sorte d'une part à bien séparer les brins 2 et d'autre part à permettre un coulissement aisé de la pièce 17 dans la gaine 4.

La pièce 17 est alors introduite partiellement dans la gaine 4 puis les brins 2 sont tendus de sorte à obtenir un bon contact entre la coiffe 14 et l'os 8.

Lors que ce bon contact est établi, la pièce 17 est enfoncée à force dans la gaine 4 afin de bloquer le déplacement des brins 2. Le coincement est réalisé d'une part entre les faces inclinées 23 des rainures 20 et la surface interne de la gaine 4 et d'autre part entre les surfaces coniques de respectivement la tête 21 et l'ouverture 22.

Cette réalisation permet d'obtenir un blocage efficace des brins 2 du fait que, lors de l'effort de tension des brins 2, la tête 21 sert de butée au mouvement de translation de la pièce 17 à l'intérieur de la gaine 4.

On décrit ci-dessous un kit d'ancrage de brins de suture à l'intérieur d'un tunnel osseux 3 qui comprend un système implantable 16 tel que décrit ci-dessus et un dispositif d'implantation 24 dudit système 16.

Suivant le mode de réalisation représenté sur les figures, le dispositif d'implantation 24 comprend :
- une pièce d'actionnement 25 de la pièce complémentaire 17 depuis sa première position vers sa deuxième position ; et
- un outil 26 agencé pour recevoir le dispositif implantable 1, les brins de suture 2, la pièce complémentaire 17 et la pièce d'actionnement 25, ledit outil 26 comprenant des moyens d'actionnement de la pièce d'actionnement 25.

La pièce d'actionnement 25 et l'outil 26 peuvent être réalisés en matériau métallique ou polymérique.

Lorsque la pièce complémentaire 17 est dans sa première position, la mise en place du système 16 à l'intérieur du tunnel osseux 3 est réalisée en le faisant coulisser sur les brins de suture 2 à fixer puis le blocage des brins de suture 2 est réalisé par actionnement de la pièce complémentaire 17.

Dans le mode de réalisation représenté sur les figures, l'outil 26 comprend un tube 27 dans lequel la pièce d'actionnement 25 est disposée coulissante, une poignée 28 et une première gâchette 29 permettant de provoquer le déplacement de la pièce d'actionnement 25 à l'intérieur du tube 27 selon une course maîtrisée.

La première gâchette 29, montée pivotante autour d'un axe 30, comprend des moyens 31 apte à pousser, selon une course maîtrisée, les pièces 17, 25 à l'intérieur du tube 27.

L'extrémité distale 32 du tube 27 correspond sensiblement à l'empreinte de la tête 5 du dispositif implantable 1 de sorte à pouvoir la loger.

Pour permettre d'une part la mise place et le passage des brins de suture 2 et d'autre part d'immobiliser le dispositif implantable 1 par clipsage, l'extrémité distale 32 du tube 27 comprend deux fentes 33 disposées de part et d'autre du tube 27.

Deux rainures 34 sont également prévues sur la face extérieure du tube 27 et dans le prolongement des deux fentes 33 de sortes à guider les brins de suture 2 à l'extérieur du tube 27.

Un dispositif de manipulation des brins est prévu au voisinage de l'extrémité proximale du tube 27 qui comprend :
- une deuxième gâchette 35 agencée sur la poignée 28 pour être actionnable conjointement à la première 29 ; et
- deux ergots 36 disposés de part et d'autre de la poignée 28 pour permettre le blocage respectif d'un brin de suture 2 par enroulement.

Ainsi, une traction maîtrisée sur les brins 2 peut être exercée par l'opérateur par actionnement de la deuxième gâchette 35 de sorte, préalablement à l'actionnement de la pièce 25, à assurer un bon contact entre la coiffe 14 et l'os 8.

En relation avec la figure 9, on décrit une pièce d'actionnement 25 de forme cylindrique dont le diamètre est sensiblement inférieur au diamètre intérieur du tube 27 et qui comprend deux gorges 37, 38 usinées radialement sur sa surface au voisinage de son extrémité proximale.

Les gorges 37, 38 sont agencées pour coopérer avec un système de blocage de la translation de la pièce d'actionnement 25 à l'intérieur du tube 27.

Le système comprend un pointeau 39 monté sur ressort 40 qui est disposé dans la poignée 28 perpendiculairement au déplacement de la pièce d'actionnement 25 de sorte à venir s'engager dans l'une des gorges 37, 38 pour bloquer la translation (voir figure 10).

Ainsi, lorsque le pointeau 38 est engagé dans la première gorge 37, les brins 2 peuvent être disposés à l'intérieur de la gaine 4 alors que la pièce 17 est déjà partiellement engagée dans la gaine 4 puis, par actionnement partiel de la première gâchette 29, le pointeau 39 est engagé dans la deuxième gorge 38 de sorte à mettre la pièce 17 dans sa première position.

L'opérateur peut alors actionner la deuxième gâchette 35 afin de tendre les brins 2 puis actionner totalement la première gâchette 29 afin de mettre la pièce 17 dans sa deuxième position.

L'extrémité distale 41 de la pièce d'actionnement 25 peut être filetée pour pouvoir, préalablement à sa disposition dans l'outil 26, lui associer la pièce complémentaire 17 en taraudant l'intérieur de la tête 21 préalablement muni d'un trou 42.

Ainsi, en cas de mauvaise manipulation, la pièce 17 peut être désengager facilement de la gaine 4.

Dans cette réalisation, la pièce complémentaire 17 peut être montée sur la pièce d'actionnement 25 préalablement à son introduction dans le tube 27 et un dispositif (non représenté) permet de désengager la pièce d'actionnement 25 de la pièce complémentaire 17 dans sa deuxième position, par exemple en prévoyant à l'extrémité proximale de la pièce d'actionnement 25 un mollette apte à la mettre en rotation.

## Revendications

1. Système implantable (16) pour l'ancrage de brins de suture (2) à l'intérieur d'un tunnel osseux (3), **caractérisé en ce qu'**il comprend un dispositif implantable (1) et une pièce complémentaire (17), ledit dispositif étant formé d'une seule pièce réalisée en matériau biorésorbable et comprend :
- une gaine (4) agencée pour recevoir au moins un brin de suture (2) ; et
- une tête (5) disposée à une extrémité proximale (6) de ladite gaine (4) et s'étendant radialement depuis la paroi extérieure (7) de celle-ci ;
ladite pièce étant destinée à être introduite dans la gaine (4), de sorte à, dans une première position, laisser libre le déplacement des brins de suture (2) à l'intérieur de la gaine (4) et, dans une deuxième position, être apte à bloquer ce déplacement.

2. Système selon la revendication 1, **caractérisé en ce que** la paroi extérieure (7) de la gaine (4) comprend des moyens d'ancrage du dispositif (1) dans le tunnel osseux (3).

3. Système selon la revendication 1 ou 2, **caractérisé en ce que** la gaine (4) est formée d'une pièce annulaire dont l'extrémité distale (11) est de forme tronconique, ladite pièce possédant une ouverture (12) de section sensiblement circulaire qui la traverse de part en part selon son axe longitudinal.

4. Système selon la revendication 3, **caractérisé en ce que** les moyens d'ancrage sont formés d'au moins une sur-épaisseur (13) en forme d'anneau qui s'étend radialement sur la surface externe (7) de la pièce annulaire.

5. Système selon l'une quelconque des revendications 1 à 4, **caractérisé en ce que** la tête (5) est de section rectangulaire, la face inférieure (9) de ladite tête (5) formant un angle sensiblement égal à 45° avec l'axe longitudinal de la gaine (4).

6. Système selon l'une quelconque des revendications 1 à 4, **caractérisé en ce que** la tête (5) est de section rectangulaire, la face inférieure (9) de ladite tête (5) formant un angle sensiblement égal à 90° avec l'axe longitudinal de la gaine (4).

7. Système selon l'une quelconque des revendications 1 à 6, **caractérisé en ce que** la pièce complémentaire (17) est formée d'une tige cylindrique (18) dont la section est légèrement inférieure au diamètre intérieur de la gaine (4) et dont la partie distale (19) est munie de rainures (20) destinées à recevoir chacune un brin de suture (2), lesdites rainures (20) étant agencées pour d'une part séparer lesdits brins (2) et d'autre part guider leur coulissement à l'intérieur de la gaine (4).

8. Système selon la revendication 7, **caractérisé en ce que** la partie distale (19) comprend deux rainures (20) s'étendant suivant la direction longitudinale et sur moins de la moitié de la longueur totale de la tige (18).

9. Système selon l'une quelconque des revendications 1 à 8, **caractérisé en ce qu'**une tête (21) est prévue sur l'extrémité proximale de la tige (18), ladite tête (21) étant destinée d'une part à participer au coincement des brins (2) lorsque la tige (18) est dans sa deuxième position et d'autre part à servir de butée lors du positionnement de la tige (18) dans ladite deuxième position.

10. Système selon la revendication 9, **caractérisé en ce que** l'ouverture proximale (22) de la gaine (4) et la tête (21) de la tige (18) sont de forme conique, le diamètre de la tête (21) étant supérieur à celui de l'ouverture proximale (22), l'angulation de la tête (21) étant sensiblement égale ou supérieure à celle de l'ouverture proximale (22) et la hauteur de la tête (21) étant sensiblement égale ou inférieure à la profondeur du cône de l'ouverture proximale (22).

11. Système selon la revendication 10, **caractérisé en ce que** les angulations de la tête (21) et de l'ouverture proximale (22) sont de l'ordre de 10°.

12. Système selon l'une quelconque des revendications 1 à 11, **caractérisé en ce que** le dispositif (1) et la pièce complémentaire (17) sont en matériau biorésorbable, identique ou non.

13. Système selon la revendication 12, **caractérisé en ce que** le matériau biorésorbable comprend les polymères de grande pureté chimique, de masse moléculaire supérieure à environ 250 000 et de faible polydispersité, par exemple inférieur à 2.

14. Système selon la revendication 13, **caractérisé en ce que** le matériau biorésorbable comprend les stéréocopolymères des acides L- et D-Lactique, les homopolymères de l'acide L-Lactique, les copolymères de l'acide lactique et d'un comonomère compatible tel que les dérivés d'alpha-hydroxy-acides, ainsi que les dérivés et/ou mélanges de ces produits.

15. Kit d'ancrage de brins de suture (2) à l'intérieur d'un tunnel osseux (3), comprenant un système implantable (16) selon l'une quelconque des revendications 1 à 14 et un dispositif d'implantation (24) dudit système (16).

16. Kit selon la revendication 15, **caractérisé en ce que** le dispositif d'implantation (24) comprend :
- une pièce d'actionnement (25) de la pièce complémentaire (17) depuis sa première position vers sa deuxième position ;
- un outil (18) agencé pour recevoir le dispositif implantable (1), les brins de suture (2), la pièce complémentaire (17) et la pièce d'actionnement (25), ledit outil (18) comprenant des moyens d'actionnement de la pièce d'actionnement (25);
de sorte à permettre, lorsque la pièce complémentaire (17) est dans sa première position, la mise en place du système (16) à l'intérieur du tunnel osseux (3) en le faisant coulisser sur les brins de suture (2) à fixer puis le blocage des brins de suture (2) par actionnement de la pièce complémentaire (17).

17. Kit selon la revendication 16, **caractérisé en ce que** l'outil (26) comprend un tube (27) dans lequel la pièce d'actionnement (25) est disposée coulissante, une poignée (28) et une première gâchette (29) permettant de provoquer le déplacement de la pièce d'actionnement (25) à l'intérieur du tube (27) selon une course maîtrisée.

18. Kit selon la revendication 17, **caractérisé en ce que** la forme de l'extrémité distale (32) du tube (27) correspond sensiblement à l'empreinte de la tête (5) du dispositif implantable (1) de sorte à pouvoir la loger.

19. Kit selon la revendication 18, **caractérisé en ce que** l'extrémité distale (32) du tube (27) comprend en outre deux fentes (33) disposées de part et d'autre du tube (27), lesdites fentes (33) étant agencées pour permettre d'une part la mise place et le passage des brins de suture (2) et d'autre part d'immobiliser le dispositif implantable (1) par clipsage.

20. Kit selon la revendication 19, **caractérisé en ce que** deux rainures (34) sont prévues sur la face extérieure du tube (27) et dans le prolongement des deux fentes (33) de sorte à guider les brins de suture (2) à l'extérieur du tube (27), un dispositif de manipulation des brins (2) étant prévu au voisinage de l'extrémité proximale du tube (27).

21. Kit selon la revendication 20, **caractérisé en ce que** le dispositif de manipulation comprend :
- une deuxième gâchette (35) agencée sur la poignée (28) pour être actionnable conjointement à la première (29) ;
- deux ergots (36) disposés de part et d'autre de la poignée (28) pour permettre le blocage respectif d'un brin de suture (2) par enroulement ;
de sorte à pouvoir exercer une traction maîtrisée sur les brins (2) par actionnement de la deuxième gâchette (35).

22. Kit selon l'une quelconque des revendications 17 à 21, **caractérisé en ce que** la pièce d'actionnement (25) est de forme cylindrique dont le diamètre est sensiblement inférieur au diamètre intérieur du tube (27) et comprend deux gorges (37, 38) usinées radialement sur sa surface au voisinage de son extrémité proximale, lesdites gorges (37, 38) étant agencées pour coopérer avec un système de blocage de la translation de la pièce d'actionnement (25) à l'intérieur du tube (27), ledit système comprenant un pointeau (39) monté sur ressort (40) qui est disposé dans la poignée (28) perpendiculairement au déplacement de la pièce d'actionnement (25) de sorte à venir s'engager dans l'une des gorges (37, 38) pour bloquer la translation.

23. Kit selon l'une quelconque des revendications 16 à 22, **caractérisé en ce que** l'extrémité distale (41) de la pièce d'actionnement (25) est filetée pour pouvoir, préalablement à sa disposition dans l'outil (26), lui associer la pièce complémentaire (17) en taraudant l'intérieur de la tête (21) préalablement trouée.
